# FASCICULE DE BREVET EUROPEEN

(11) **EP 1 720 582 B1**
(45) Date de publication et mention de la délivrance du brevet: **06.08.2008**
(21) Numéro de dépôt: 05706542.7
(22) Date de dépôt: 02.03.2005
(51) Int. Cl.: A61M 1/00, A61M 3/02

(54) **Cassette pour une machine d'irrigation ou d'aspiration utilisée en endoscopie**
Kassette für ein Irrigations- oder Aspirationsgerät für die Endoscopie
Cassette for an irrigation or aspiration machine for endoscopy

(30) Priorité: 05.03.2004 EP 04100917; 18.06.2004 FR 0406620
(43) Date de publication de la demande: 15.11.2006
(60) Demande divisionnaire: 07020099.3
(73) Titulaire: Future Medical System S.A., 1217 Meyrin (CH)
(72) Inventeur: TACHOIRE, Raphaël, 06700 Saint Laurent du Var (FR); CHAUTARD, Stanislas, 92200 Neuilly sur Seine (FR); FRANCISCO, André, 06560 Sophia Antipolis (FR); JANIN, Steven, 06200 Nice (FR); RODRIGUEZ, Christian, 06640 Saint Jeannet (FR); JANIN, Patrick, 06000 Nice (FR); PASCUAL, Thierry, 06700 Saint Laurent du Var (FR); DIAS, Armando, 06300 Nice (FR)
(74) Mandataire: Savoye, Jean-Paul
(86) Numéro de dépôt international: PCT/CH2005/000123
(87) Numéro de publication internationale: WO 2005/084728

(56) Documents cités:
- US-A- 5 246 422
- US-A- 5 460 490
- US-A- 5 626 563
- US-A- 5 628 731
- US-A- 5 649 905
- US-A1- 2002 147 423
- US-A1- 2003 229 302

## Description

### Domaine technique

L'invention se rapporte à une cassette destinée à s'insérer dans une machine d'irrigation ou d'aspiration utilisée en endoscopie.

L'invention se rapporte plus particulièrement à une cassette comprenant une tubulure d'irrigation ou une tubulure d'aspiration et un support muni d'une ou de deux prises d'entrée et d'une ou de deux prises de sortie, la ou les deux tubulures formant un coude pour s'engager avec la ou les deux prises d'entrée et de sortie suivant un sens de circulation respectivement entrant et sortant et formant un segment de pompage d'irrigation ou d'aspiration suivant le sens entrant de circulation.

### Etat de la technique

Une cassette de ce type est connue du document US 5460490.

Selon ce document, le segment de pompage est prévu sur la tubulure d'irrigation. La tubulure d'aspiration ne comprend pas de segment de pompage et doit être reliée à une source à dépression pour créer l'aspiration. Cet agencement présente l'inconvénient de choisir pour le segment de pompage, le coude formé par la tubulure d'irrigation par rapport au support. De ce fait, le coude reste accessible et n'est pas protégé par le support contre un risque d'arrachement de la tubulure d'irrigation suite à une mauvaise manipulation de la cassette.

Une cassette assurant une meilleure protection des tubulures d'irrigation et d'aspiration est connue du document US 5628731. Selon ce document, le support possède une base et un couvercle se refermant sur la base pour protéger les deux tubulures. Toutefois, il est nécessaire d'ouvrir des lumières dans la base et dans le couvercle pour dégager le segment de pompage formé sur la tubulure d'irrigation.

De surcroît, les tubulures d'irrigation et d'aspiration sont disposées en imbrication l'une dans l'autre sur un même plan de la base du support. Cet agencement tend à imposer un encombrement d'autant plus important de la cassette qu'une deuxième tubulure d'aspiration, en général souhaitée par le chirurgien utilisateur de la cassette, doit être engagée sur le support.

Le but de l'invention est de modifier une cassette conforme à celle qui vient d'être décrite ci-dessus pour garantir une bonne protection des tubulures d'irrigation et d'aspiration tout en réduisant le nombre de pièces nécessaires au support pour l'engagement des tubulures et en permettant d'engager une tubulure d'aspiration dérivée sans augmenter l'encombrement général de la cassette.

### Divulgation de l'invention

A cet effet, l'invention a pour objet une cassette destinée à s'insérer dans une machine d'irrigation ou d'aspiration utilisée en endoscopie comprenant une tubulure d'irrigation ou une tubulure d'aspiration et un support muni d'une ou de deux prises d'entrée et d'une ou de deux prises de sortie, la ou les deux tubulures formant un coude pour s'engager avec la ou les deux prises d'entrée et de sortie suivant un sens de circulation respectivement entrant et sortant et formant un segment de pompage d'irrigation ou d'aspiration suivant le sens entrant de circulation, caractérisée en ce que le support comprend un guide en T conformé à la tête du T pour protéger le coude de chaque tubulure et conformé le long du corps du T en un logement guidant la ou les deux tubulures suivant le sens de circulation sortant, le guide en T s'étendant entre la ou les deux prises d'entrée pour former le segment de pompage d'irrigation ou d'aspiration de part et d'autre du logement entre chaque prise d'entrée et la tête du T.

Le guide en T permet de protéger la ou les deux tubulures dans les deux sens de circulation entrant et sortant sans qu'il soit nécessaire de prévoir un couvercle au support. Le logement guide la ou les deux tubulures, en permettant de les superposer l'une par rapport à l'autre pour réduire l'encombrement de la cassette par comparaison à une disposition de deux tubulures dans un même plan. En s'étendant entre les deux prises d'entrée des tubulures, le guide en T permet de surcroît de former un segment de pompage pour chacune des deux tubulures d'irrigation et d'aspiration.

### Brève description des dessins

D'autres avantages de l'invention apparaîtront à la lecture de la description d'un mode de réalisation illustré ci-après par les dessins.

La figure 1 montre en perspective une cassette selon l'invention.
La figure 2 montre la cassette en vue de face.
La figure 3 montre la cassette suivant une coupe A-A.
La figure 4 montre la casette suivant une coupe B-B.
La figure 5 montre la casette suivant une vue de dessous.
La figure 6 montre la cassette suivant une coupe C-C.
La figure 7 montre la casette suivant une coupe D-D.
La figure 8 montre un autre exemple de réalisation d'une cassette selon l'invention.
La figure 9 montre la cassette de la figure 8 en vue de dessous.
La figure 10 montre la cassette de la figure 8 suivant une coupe F-F.
La figure 11 montre un exemple de réalisation d'une cassette selon la figure 8, dans lequel on a retiré la fonction d'aspiration pour ne laisser que la fonction d'irrigation.
La figure 12 montre la cassette de la figure 11 en vue de dessous.
La figure 13 montre en perspective une machine d'irrigation et d'aspiration dans laquelle est insérée une cassette selon l'invention. La figure 14 montre en vue de dessus un chariot et un porte-cassette de la machine illustrée par la figure 13.
La figure 15 est une vue de côté de la figure 14.
La figure 16 est une vue selon la coupe H-H de la figure 14.
La figure 17 montre plus particulièrement le chariot de la figure 13.
La figure 18 montre plus particulièrement le porte-cassette de la figure 13.

**Tableau 1**

| | |
|---|---|
| 1i,1a | Tubulure d'irrigation, d'aspiration |
| 3i,3a | Prises d'entrée |
| 5 | Support |
| 7i,7a | Prises de sortie |
| 9i,9a | Coudes |
| 10i,10a | Pièces tubulaires d'irrigation, d'aspiration |
| 11i,11a | Segment de pompage d'irrigation, d'aspiration |
| 13 | Guide en T |
| 14 | Capot de protection |
| 15i,15a | Extrémités d'entrée |
| 17 | Arrondi |
| 19 | Logement |
| 21 | Boîtier |
| 23i,23a | Canaux d'entrée |
| 25 | Troisième canal d'entrée |
| 26 | Extrémité |
| 27 | Supports en forme de demi-disques |
| 28 | Voie de communication |
| 29i,29a | Canaux de sortie |
| 31 | Chambre |
| 33,35 | Tubulures d'aspiration complémentaires |
| 36 | Paroi de fond |
| 37,39 | Tubulures d'irrigation complémentaires |
| 41 | Tubulure d'aspiration complémentaire |
| 43 | Chambre |
| 45 | Paroi de fond |
| 47 | Prises de pression |
| 48 | Pièce en élastomère |
| 49 | Lignes de pression |
| 51i,51a | Pompe péristaltique d'irrigation, d'aspiration |
| 53i,53a | Sabots |
| 54i,54a | Roues |
| 55i,55a | Galets |
| 61 | Châssis |
| 62 | Ressorts de compression |
| 63 | Chariot |
| 64 | Butée |
| 65 | Porte-cassette |
| 66 | Capteur de position |
| 67a,69a | Obturateurs |
| 68 | Capteurs de position |
| 71 | Troisième obturateur |
| 73 | Moyen de verrouillage |
| 75 | Butée |
| 77 | Doigts de reconnaissance |
| 79 | Moyen de centrage |
| 83 | Glissières |
| 85 | Actionneur linéaire |
| 86 | Tige |
| 87 | Glissières |
| 88 | Capteurs de position |
| 89 | Glissières |
| 90 | Capteurs de position |
| 91 | Ressorts |
| 93 | Glissières |
| 94 | Actionneurs linéaires |
| 95 | Tétines |
| 96 | Actionneur linéaire |

### Mode(s) de réalisation de l'invention

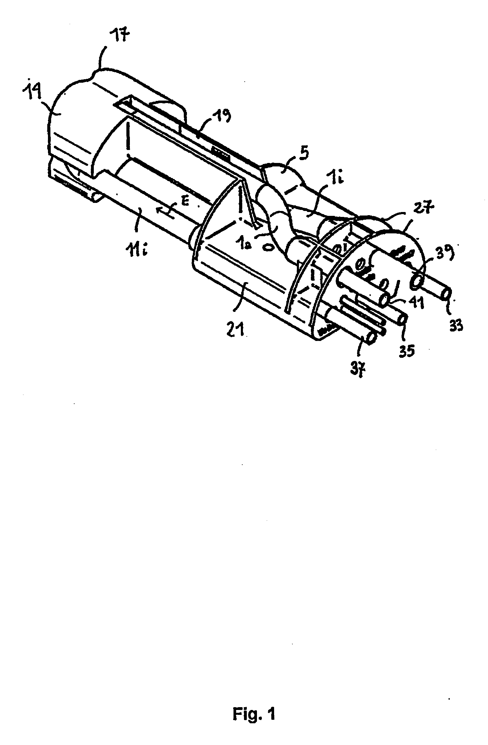

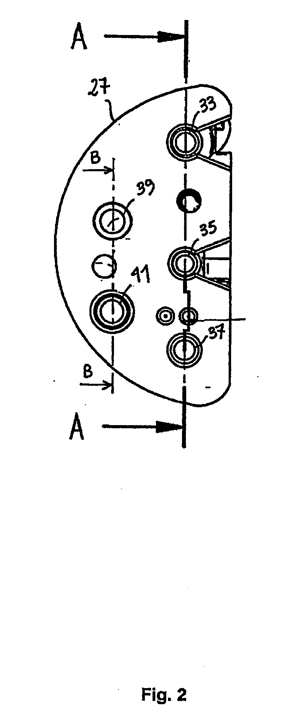

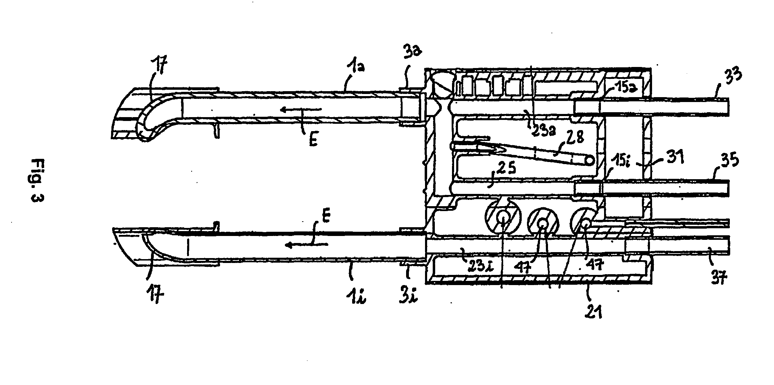

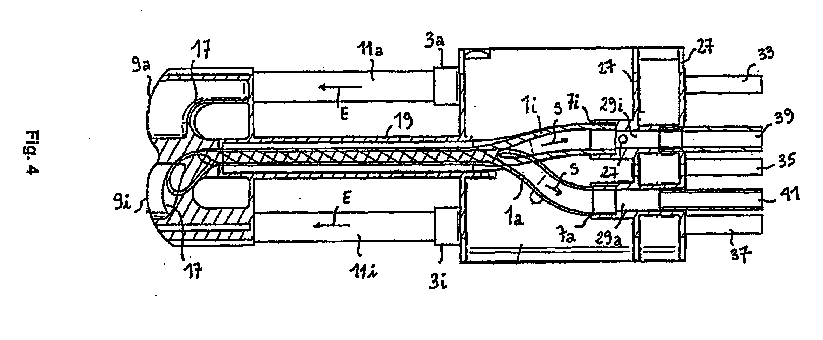

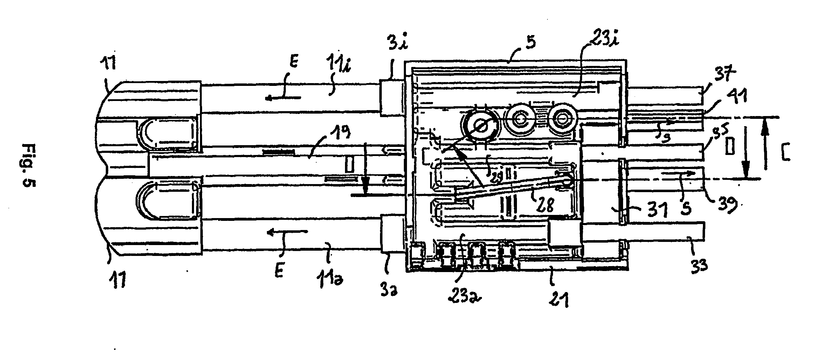

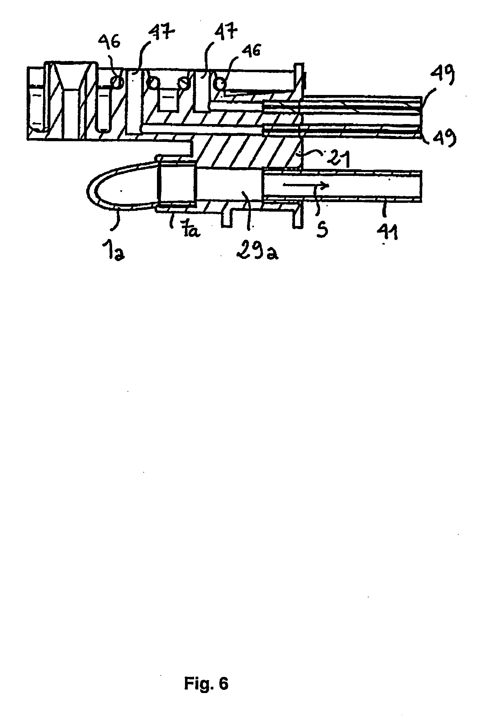

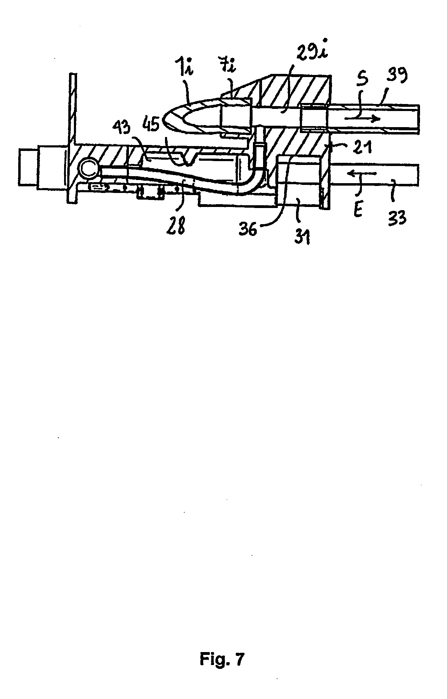

En référence aux figures 1 à 7, une cassette destinée à s'insérer dans une machine d'irrigation et d'aspiration utilisée en endoscopie comprend une tubulure d'irrigation 1i, une tubulure d'aspiration 1a et un support 5 muni de deux prises d'entrée 3i,3a et de deux prises de sortie 7i,7a. Les deux tubulures forment l'une et l'autre un coude 9i,9a pour s'engager avec les deux prises d'entrée et de sortie suivant un sens de circulation respectivement entrant E et sortant S et forment l'une ou l'autre un segment de pompage d'irrigation 11i ou d'aspiration 11a suivant le sens entrant E de circulation.

Selon l'invention, le support 5 comprend un guide en T 13 conformé à la tête du T pour protéger le coude 9i,9a de chaque tubulure 1i,1a et conformé le long du corps du T en un logement 19 guidant les deux tubulures superposées l'une 1i par rapport à l'autre 1a suivant le sens de circulation sortant S.

Dans l'exemple illustré par les figures 1 à 7, la tête du T comprend un capot de protection 14 et forme un double arrondi 17 pour guider les deux coudes 9i,9a de chaque tubulure.

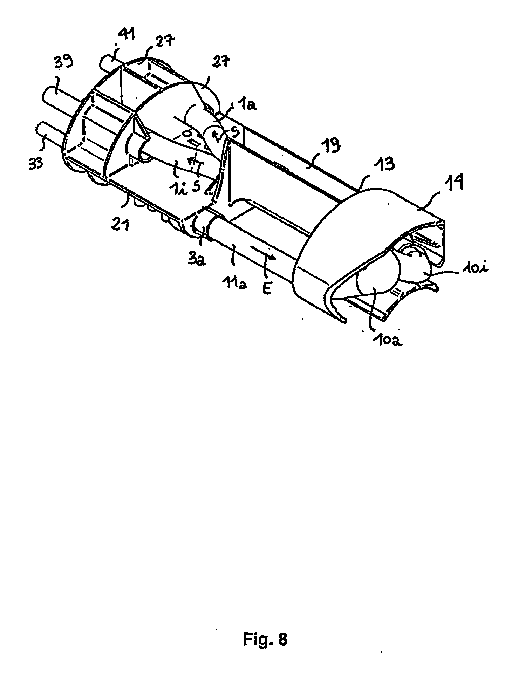

Dans l'exemple de la figure 8, la tête du T comprend là encore un capot de protection 14 mais les coudes 9i,9a sont formés par des pièces tubulaires 10i,10a fabriquées dans une matière plastique souple et insérées entre deux portions de chaque tubulure d'irrigation et d'aspiration.

Le guide en T 13 s'étend entre les deux prises d'entrée 3i,3a pour former le segment de pompage d'irrigation 11i ou d'aspiration 11a de part et d'autre du logement 19 entre chaque prise d'entrée 3i,3a et la tête du T.

Le guide en T 13 est fixé à un boîtier 21 intégré au support 5 et pourvu de deux canaux d'entrée 23i,23a ouverts à une extrémité d'entrée 15i,15a et débouchant à une extrémité opposée par les prises d'entrée 3i,3a pour assurer une communication avec les deux tubulures 1i,1a suivant le sens de circulation entrant E.

Le boîtier 21 est pourvu d'un troisième canal d'entrée 25 ouvert à une extrémité 26 et disposé en dérivation par rapport au canal d'entrée 23a communiquant avec la tubulure d'aspiration 1a pour déboucher, à une extrémité opposée, par la prise d'entrée 3a assurant la communication avec la tubulure d'aspiration 1a.

Le canal d'entrée 23a communiquant avec la tubulure d'aspiration 1a et le troisième canal d'entrée 25 monté en dérivation s'ouvrent, à l'extrémité opposée à la prise d'entrée 3a assurant la communication avec la tubulure d'aspiration 1a, dans une chambre 31 intégrée au boîtier et recevant deux tubulures d'aspiration complémentaires 33,35 s'engageant avec ces deux canaux 23a,25 en étant disposées à distance d'une paroi de fond 36 de la chambre 31 pour être comprimées contre cette paroi de fond 36 dans une position d'obstruction de ce canal d'entrée 23a communiquant avec la tubulure d'aspiration 1a ou de ce troisième canal d'entrée 25.

Le boîtier 21 est pourvu de deux canaux de sortie 29i,29a ouverts à une extrémité de sortie et débouchant à une extrémité opposée par les prises de sortie 7i,7a pour assurer une communication avec les deux tubulures 1i,1a suivant le sens de circulation sortant S.

Les canaux de sortie 29i,29a sont portés par des supports en forme de demi-disques 27 s'étendant dans un plan perpendiculaire à un plan du boîtier pour être surélevés par rapport aux canaux d'entrée 23i,23a,25.

Le boîtier 21 est pourvu d'une voie de communication 28 entre le canal de sortie 29i communiquant avec la tubulure d'irrigation 1i et le canal d'entrée 23a communiquant avec la tubulure d'aspiration 1a ou le troisième canal d'entrée 25 monté en dérivation par rapport à ce dernier.

La voie de communication 28 est assurée par une tubulure disposée dans une chambre 43 intégrée au boîtier 21 et à distance d'une paroi de fond 45 de cette chambre pour être comprimée contre cette paroi de fond dans une position d'obstruction de cette voie de communication.

Dans l'exemple illustré par les figures 1 à 7, le boîtier 21 incorpore une ou deux prises de pression 47 formées de conduits pour le passage d'air provenant de lignes de pression 49 branchées sur une tubulure d'irrigation complémentaire 39 par l'intermédiaire d'un détecteur de pression à membrane.

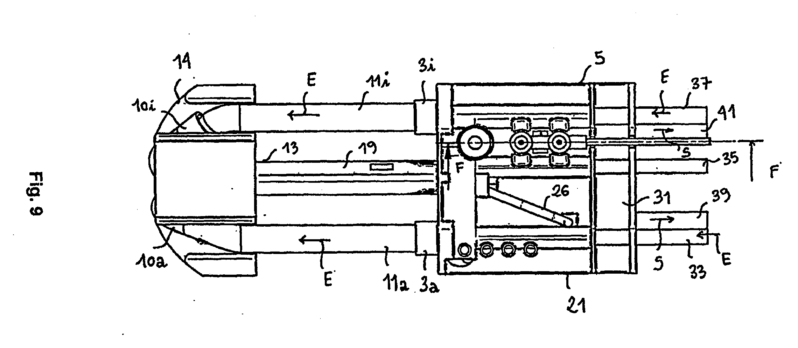

Dans l'exemple illustré par la figure 9, le boîtier 21 est prévu pour recevoir une pièce en élastomère 48 formant d'une seule pièce, les deux prises de pression 47 et les conduits pour le passage d'air provenant des lignes de pression 49.

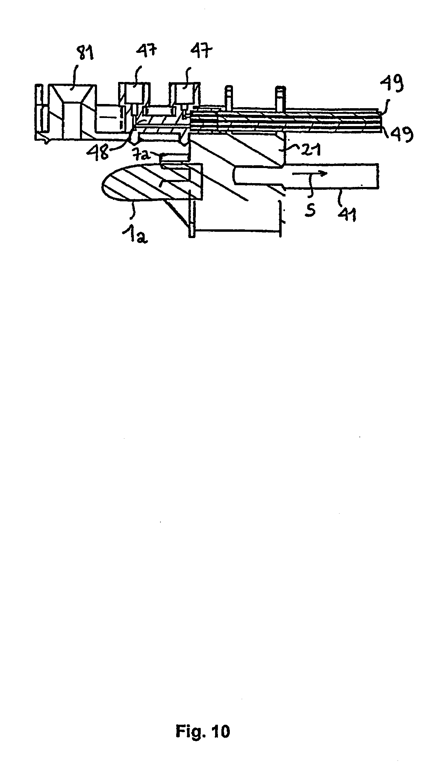

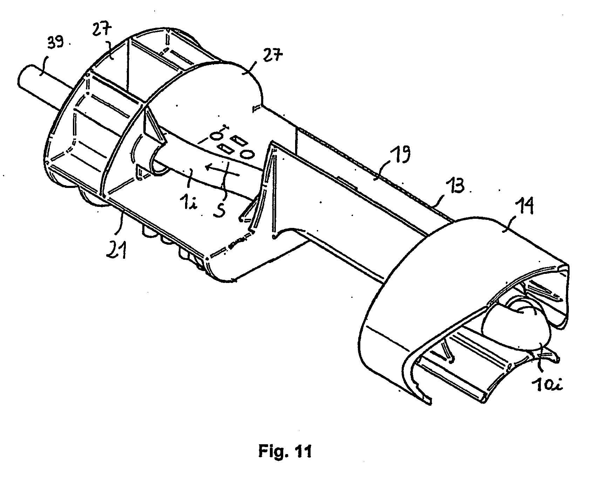

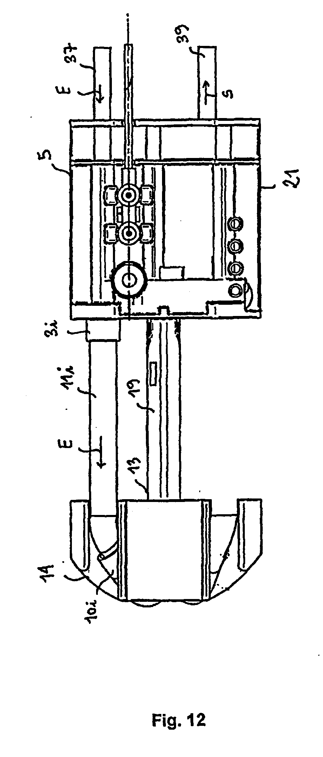

Dans l'exemple illustré par les figures 11 et 12, ont été retirés par rapport à l'exemple de réalisation illustré par les figures 8 à 10, la tubulure d'aspiration 1a, la prise d'entrée 3a et le segment de pompage d'aspiration 11a. Dans le boîtier 21, ont été également retirés le canal d'aspiration 23a, le canal de sortie 29a ainsi que les tubulures d'aspiration complémentaires 33,35 et 41. Dans cet exemple de réalisation, la cassette selon l'invention ne permet de réaliser que irrigation et convient plus particulièrement pour une utilisation en endoscopie à des fins de diagnostic.

Une machine d'irrigation et d'aspiration à cassette utilisée en endoscopie est maintenant décrite. Cette machine ne fait pas partie de l'invention.

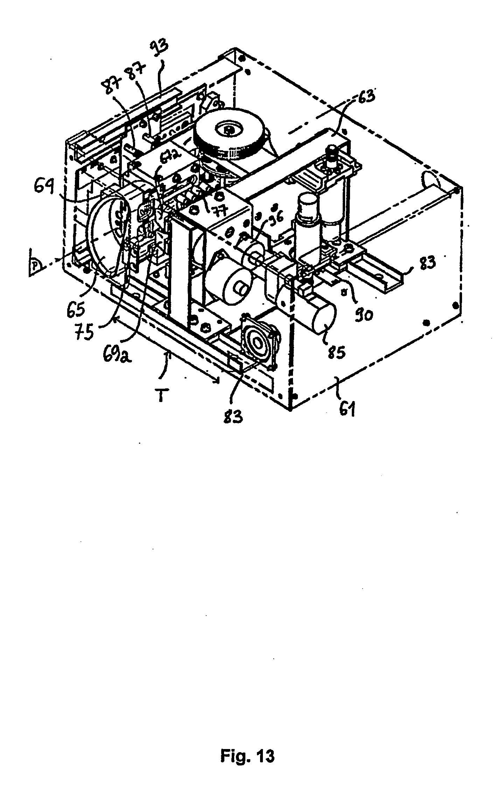

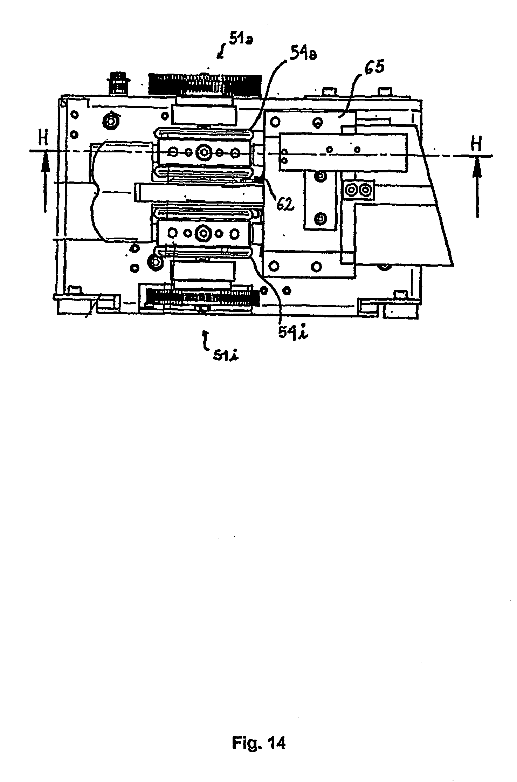

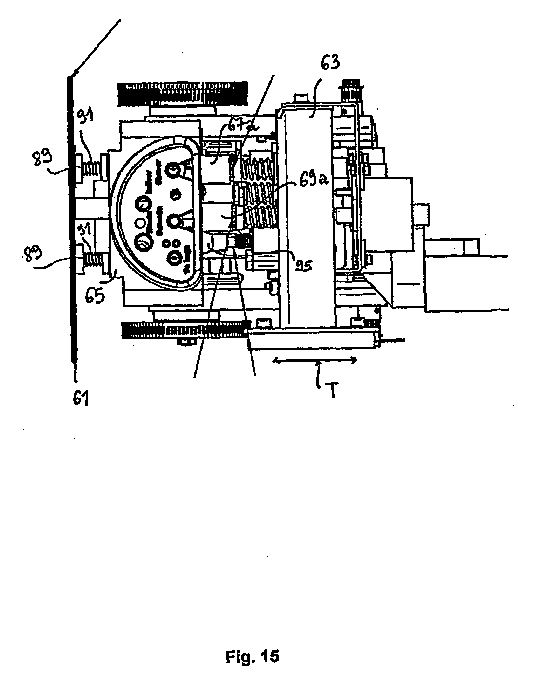

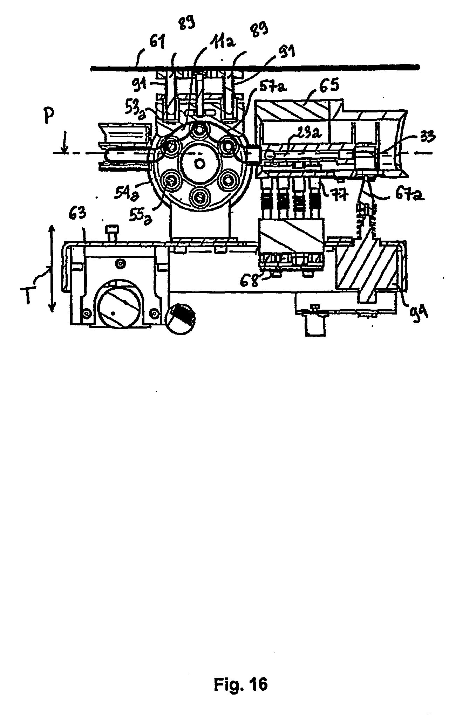

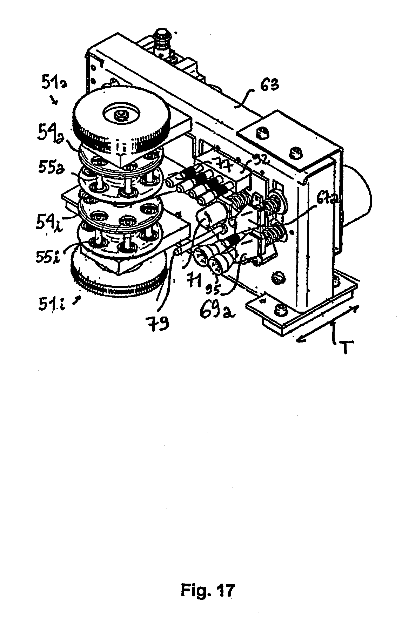

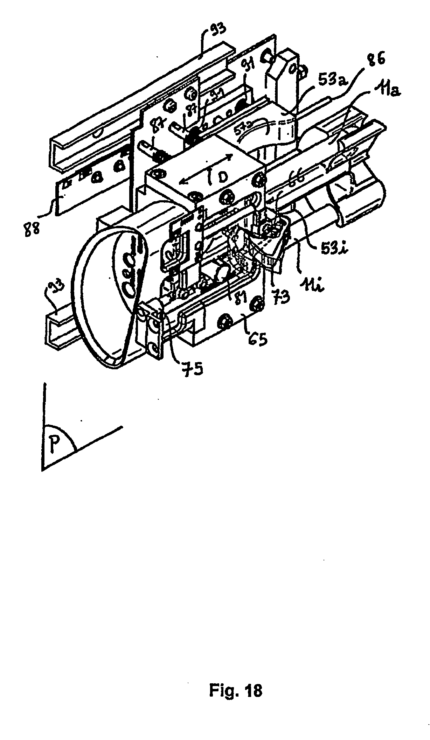

En référence aux figures 13 à 18, la machine comprend une pompe péristaltique d'irrigation 51i à sabot 53i et à roue 54i à galets 55i montés en correspondance, l'un 53i sur un châssis 61 et l'autre 54i sur un chariot 63 mobile le long de glissières 83 fixées au châssis 61 pour s'étendre suivant une direction de translation T. Un actionneur linéaire 85 commande la translation du chariot 63 entre une position de repos par desserrement du sabot 53i par rapport aux galets 55i et une position de pompage par resserrement du sabot 53i par rapport aux galets 55i. La machine comprend également un porte-cassette 65 monté sur le châssis 61 pour s'étendre dans un plan P perpendiculaire à la direction de translation T et passant entre le sabot 53i et la roue 54i à galets 55i de la pompe d'irrigation 51i.

La machine comprend une pompe péristaltique d'aspiration 51a à sabot 53a et à roue 54a à galets 55a montés en correspondance, l'un 53a sur le châssis 61 et l'autre 54a sur le chariot 63 pour desserrer ou resserrer ledit sabot 53a par rapport audits galets 55a suivant la direction de translation T lors du desserrement ou du resserrement du sabot 53i par rapport aux galets 55i de la pompe péristaltique d'irrigation 51i entre la position de repos et la position de pompage, le plan P perpendiculaire à la direction de translation T passant également entre le sabot 53a et la roue 54a à galets 55a de la pompe d'aspiration.

L'agencement du porte-cassette 65 dans un plan perpendiculaire à la direction de translation T du chariot mobile 63 permet de disposer à la fois le segment de pompage d'irrigation 11i et le segment de pompage d'aspiration 11a d'une cassette selon l'invention entre les sabots 53i,53a et les galets 55i,55a respectivement de la pompe péristaltique d'irrigation 51i et la pompe péristaltique d'aspiration 51a. La cassette selon l'invention est introduite dans le porte-cassette 65 lorsque les sabots sont desserrés par rapport aux galets dans la position de repos pour que les segments de pompage d'irrigation 11i et d'aspiration 11a soient ensuite laminés entre les sabots et les galets dans la position de pompage.

De préférence, le porte-cassette 65 est monté mobile par rapport au châssis 61 pour être entraîné en déplacement par le chariot 63 lorsque ce dernier est déplacé de la position de repos à la position de pompage. Le déplacement du porte-cassette 65 s'effectue le long de glissières 87 fixées au châssis 61 parallèlement à la direction de translation T. Avantageusement, les sabots 53i,53a des pompes péristaltiques d'irrigation 51i et d'aspiration 51a sont montés mobiles par rapport au châssis 65 le long de glissières 89 s'étendant parallèlement à la direction de translation T. Le déplacement des sabots 53i,53a s'effectue contre la compression de ressorts 91 disposés autour des glissières 89 pour régler une pression de laminage entre les sabots 53i,53a et les galets 55i,55a lorsque le chariot 63 s'est déplacé dans la position de pompage.

Pour faciliter l'introduction ou le retrait d'une cassette selon l'invention dans la machine d'irrigation et d'aspiration, le porte-cassette 65 est monté mobile le long de glissières 93 fixées au châssis 61 pour être déplacé parallèlement au plan P perpendiculaire à la direction de translation T. Le déplacement du porte-cassette 65 est commandé par un actionneur linéaire entre une position d'insertion de cassette où le porte-cassette 65 s'est rapproché des sabots et des roues à galets des pompes d'irrigation et d'aspiration et une position d'éjection de casette où le porte-cassette 65 s'est éloigné desdits sabots et desdites roues à galets.

La machine d'irrigation et d'aspiration à cassette comprend en outre un moyen de centrage 79 monté sur le chariot 63 pour être déplacé avec le chariot 63 suivant la direction de translation T depuis la position de repos vers la position de pompage et après que le porte-cassette 65 se soit rapproché des sabots 53i,53a et des roues 54i,54a à galets des deux pompes d'irrigation et d'aspiration dans la position d'insertion de cassette.

### Application industrielle,

Le support 5 d'une cassette selon l'invention ainsi que le guide en T 13 avec le capot de protection 14 à la tête du T et le logement 21 le long du corps du T, le boîtier intégré au support 21 pourvu des canaux d'entrée 23i,23a, le troisième canal d'entrée 25, les prises d'entrée 3i,3a, les prises de sortie 7i,7a et les supports 27 sont d'une seule pièce de préférence moulée par injection de matière plastique.

L'utilisation de la cassette selon l'invention et de la machine d'irrigation et d'aspiration s'effectue de la façon suivante. La cassette est introduite à la main dans le porte-cassette 65 jusqu'à ce qu'un moyen de verrouillage 73 monté en pivot par rapport au porte-cassette 65 vienne verrouiller le support 5 de la cassette par rapport au porte-cassette 65 contre la compression de ressorts 62 portés par le porte-cassette. Le pivotement du moyen de verrouillage 73 est détecté par un capteur de position 66 fixé au porte-cassette 65.

Il convient de noter que le capot de protection 14 et les supports en forme de demi-disques 27 offrent une fonction pour détromper un utilisateur lors de l'insertion de la cassette dans le porte-cassette 65.

L'actionneur linéaire commandant le déplacement du porte-cassette 65 déplace ce dernier dans la position d'insertion de cassette pour disposer les segments de pompage d'irrigation 11i et d'aspiration 11a des tubulures d'irrigation 1i et d'aspiration 1a entre les sabots 53i,53a et les roues 54i,54a à galets respectivement de la pompe d'irrigation et de la pompe d'aspiration. Des capteurs de position 88 sont fixés au châssis 61 pour contrôler le déplacement du porte-cassette 65 dans le plan P perpendiculaire à la direction de translation T. Une tige 86 est fixée à l'actionneur linéaire commandant le déplacement du porte-cassette 65 pour venir en butée contre le châssis arrêter le déplacement du porte-cassette dans la position d'insertion de la cassette.

L'actionneur linéaire 85 commandant le déplacement du chariot mobile 63 déplace ensuite ce dernier suivant la direction de translation T de la position de repos à la position de pompage pour entraîner en déplacement le porte-cassette 65 et venir presser les segments de pompage d'irrigation 1i et d'aspiration 1a de la cassette entre les sabots et les galets respectivement de la pompe d'irrigation et de la pompe d'aspiration. Des capteurs de position 90 sont fixés au châssis 61 pour contrôler le déplacement du chariot 63 suivant la direction de translation T. Les sabots 53i,53a des pompes péristaltiques d'irrigation et d'aspiration se déplacent contre la compression des ressorts 91 disposés autour des glissières 87 pour régler une pression de laminage entre les sabots 53i,53a et les galets 55i,55a lorsque le chariot 63 s'est déplacé dans la position de pompage. Le moyens de centrage 79 porté par le chariot mobile 63 s'insère dans un moyen de centrage 81 porté par le boîtier 21 de la cassette pour centrer la cassette par rapport au chariot 63 dans la position de pompage.

Avantageusement, le chariot 63 porte des doigts de reconnaissance de cassette 77 mobiles par rapport au chariot suivant la direction de translation T. Les doigts de reconnaissance de cassette 77 sont déplacés par le chariot parallèlement à la direction de translation T pour coopérer avec des repères correspondants formés dans le boîtier 21 de la cassette pour reconnaître cette dernière dans la position de pompage et ainsi prérégler certains paramètres de fonctionnement de la machine d'irrigation et d'aspiration, notamment la vitesse de rotation de la roue à galets de la pompe d'irrigation. Des capteurs de position 68 sont prévus sur le châssis 63 pour détecter la présence ou l'absence des doigts de reconnaissance de cassette 77 et ainsi identifier la cassette insérée dans la machine d'irrigation et d'aspiration.

La tubulure d'irrigation 1i est alimentée en fluide physiologique à partir d'un réservoir et d'une tubulure d'irrigation complémentaire 37 s'engageant dans le canal d'entrée 3i du boîtier 21. Le segment de pompage 11i de la tubulure d'irrigation 1i coopère avec la pompe péristaltique d'irrigation 51i disposée dans la machine d'irrigation et d'aspiration dans laquelle la cassette est insérée pour mettre en circulation le fluide physiologique dans la tubulure d'irrigation complémentaire 39 s'engageant dans le canal de sortie 29i communiquant avec la tubulure d'irrigation 1i et débouchant dans une canule d'endoscope placée dans une zone d'intervention chirurgicale d'un patient, par exemple une articulation du genou ou de l'épaule.

Le segment de pompage 11a de la tubulure d'aspiration 1a coopère avec la pompe péristaltique d'aspiration 51a disposée dans la machine d'irrigation et d'aspiration pour aspirer, dans la tubulure d'aspiration 1a, un fluide provenant soit d'une canule soit d'un autre outil de chirurgie, par exemple un « shaver », par l'intermédiaire respectivement des tubulures d'aspiration complémentaires 33 et 35, l'une étant comprimée pour être obstruée lorsque l'autre est en service. A cet effet, le chariot 63 porte deux obturateurs 67a,69a mobiles par rapport au chariot 63 suivant la direction de translation T. Des actionneurs linéaires 94 commandent en déplacement l'un ou l'autre des obturateurs pour comprimer l'une ou l'autre des tubulures d'aspiration complémentaires 33,35 contre la paroi de fond 37 de la chambre 31 intégrée au boîtier 21 de la cassette dans la position d'obstruction du canal d'entrée 23a communiquant avec la tubulure d'aspiration 1a ou dans la position d'obstruction du troisième canal d'entrée 25 monté en dérivation par rapport à ce canal d'entrée 23a.

Le fluide aspiré circule vers un réceptacle extérieur à la machine d'irrigation et d'aspiration par l'intermédiaire d'une tubulure d'aspiration complémentaire 41 s'engageant dans le canal de sortie 29a communiquant avec la tubulure d'aspiration 1a.

La voie de communication 28 entre le canal de sortie 29i communiquant avec la tubulure d'irrigation 1i et le canal d'entrée 23a communiquant avec la tubulure d'aspiration 1a ou le troisième canal d'entrée 25 monté en dérivation par rapport à ce dernier est commandée d'une position d'obstruction à une position de circulation pour gérer une surpression accidentelle dans l'articulation du patient. A cet effet, le chariot mobile 63 porte un troisième obturateur 71 mobile par rapport au chariot 63 suivant la direction de translation T. Un actionneur linéaire 96 commande en déplacement le troisième obturateur pour comprimer la tubulure de communication 28 contre la paroi de fond 45 de la chambre 43 intégrée au boîtier 21 dans la position d'obstruction de cette tubulure de communication 28.

Avantageusement, le chariot mobile 63 porte une ou deux prises de pression d'air formées de tétines 95 communiquant avec des capteurs de pression fixés par exemple au châssis 61 pour déterminer la pression détectée par exemple sur la tubulure complémentaire d'irrigation 39 par un détecteur à membrane. Les tétines 95 sont disposées sur le chariot mobile 63 pour s'insérer dans les prises de pression 47 incorporées au boîtier 21 de la cassette selon l'invention lorsque le chariot mobile 63 s'est déplacé dans la position de pompage. Un joint 46 est monté autour des prises de pression 47 incorporées au boîtier pour assurer un contact étanche à l'air avec les tétines dans la position de pompage du chariot mobile 63.

En fin d'utilisation, l'actionneur linéaire 85 commandant le déplacement du chariot mobile 63 déplace ce dernier suivant la direction de translation T de la position de pompage à la position de repos pour desserrer les segments de pompage d'irrigation 1i et d'aspiration 1a entre les sabots et les galets respectivement de la pompe d'irrigation et de la pompe d'aspiration. Les capteurs de position 90 fixés au châssis 61 contrôlent le déplacement du chariot 63 suivant la direction de translation T. Une butée est fixée au châssis 61 pour arrêter le déplacement du chariot dans la position de repos.

L'actionneur linéaire commandant le déplacement du porte-cassette 65 déplace ensuite ce dernier dans la position d'éjection de cassette pour éloigner les segments de pompage d'irrigation 11i et d'aspiration 11a des sabots 53i,53a et des roues 54i,54a à galets respectivement de la pompe d'irrigation et de la pompe d'aspiration. Les capteurs de position 88 fixés au châssis 61 contrôlent le déplacement du porte-cassette 65 dans le plan P perpendiculaire à la direction de translation T. Une butée 64 est fixée au châssis pour arrêter le déplacement du porte-cassette dans la position d'éjection de cassette.

Pour retirer à la main la cassette de la machine d'irrigation et d'aspiration, le moyen de verrouillage 73 monté en pivot par rapport au porte-cassette 65 est actionné par une butée 75 fixée au châssis 63 pour pivoter par rapport au porte-cassette 65 lorsque ce dernier se déplace parallèlement au plan P perpendiculaire à la direction de translation T, de la position d'insertion de cassette à la position d'éjection de cassette. Les ressorts de compression 62 portés par le porte-cassette éjectent alors la cassette du porte-cassette. La machine d'irrigation et d'aspiration est prête à être utilisée avec une nouvelle cassette.

## Revendications

1. Cassette destinée à s'insérer dans une machine d'irrigation ou d'aspiration utilisée en endoscopie comprenant une tubulure d'irrigation (1 i) ou une tubulure d'aspiration (1a) et un support (5) muni d'une (3i) ou de deux prises d'entrée (3i, 3a) et d'une (7i) ou de deux prises de sortie (7i, 7a), la ou les deux tubulures formant un coude (9i, 9a) pour s'engager avec la ou les deux prises d'entrée et de sortie suivant un sens de circulation respectivement entrant (E) et sortant (S) et formant un segment de pompage d'irrigation (11 i) ou d'aspiration (11 a) suivant le sens entrant (E) de circulation, **caractérisée en ce que** le support (5) comprend un guide en T (13) conformé à la tête du T pour protéger le coude (9i, 9a) de chaque tubulure (1i, 1a) et conformé le long du corps du T en un logement (19) guidant la ou les deux tubulures suivant le sens de circulation sortant (S), le guide en T (13) s'étendant entre la ou les deux prises d'entrée (3i, 3a) pour former le segment de pompage d'irrigation (11 i) ou d'aspiration (11 a) de part et d'autre du logement (19) entre chaque prise d'entrée (3i, 3a) et la tête du T.

2. Cassette selon la revendication 1, **caractérisée en ce que** la tête du T comprend un capot de protection (14) du coude (9i, 9a) de chaque tubulure.

3. Cassette selon la revendication 1 ou 2, **caractérisée en ce que** la tête du T comprend un double arrondi (17) pour guider le coude (9i, 9a) de chaque tubulure.

4. Cassette selon la revendication 1, **caractérisée en ce que** le guide en T (13) est fixé à un boîtier (21) intégré au support (5) et pourvu d'un (23i) ou de deux canaux d'entrée (23i, 23a) ouverts à une extrémité d'entrée (151, 15a) et débouchant à une extrémité opposée par la (3i) ou les prises d'entrée (3i, 3a) pour assurer une communication avec la (1i) ou les deux tubulures (1i, 1a) suivant le sens de circulation entrant (E).

5. Cassette selon la revendication 4, **caractérisée en ce que** le boîtier (21) est pourvu d'un troisième canal d'entrée (25) ouvert à une extrémité (26) et disposé en dérivation par rapport au canal d'entrée (23a) communiquant avec la tubulure d'aspiration (1a) pour déboucher, à une extrémité opposée, par la prise d'entrée (3a) assurant la communication avec la tubulure d'aspiration (1a).

6. Cassette selon la revendication 5, **caractérisée en ce que** le canal d'entrée (23a) communiquant avec la tubulure d'aspiration (1a) et le troisième canal d'entrée (25) monté en dérivation s'ouvrent, à l'extrémité opposée à la prise d'entrée (3a) assurant la communication avec la tubulure d'aspiration (1a), dans une chambre (31) intégrée au boîtier et recevant deux tubulures d'aspiration complémentaires (33,35) s'engageant avec ces deux canaux (23a, 25) en étant disposées à distance d'une paroi de fond (36) de la chambre (31) pour être comprimées contre cette paroi de fond (36) dans une position d'obstruction de ce canal d'entrée (23a) communiquant avec la tubulure d'aspiration (1 a) ou de ce troisième canal d'entrée (25).

7. Cassette selon la revendication 4,5 ou 6, **caractérisée en ce que** le boîtier (21) est pourvu d'un (29i) ou de deux canaux de sortie (29i, 29a) ouverts à une extrémité de sortie et débouchant à une extrémité opposée par la (7i) ou les prises de sortie (7i, 7a) pour assurer une communication avec la (1i) ou les deux tubulures (1i, 1a) suivant le sens de circulation sortant (S).

8. Cassette selon la revendication 7, **caractérisée en ce que** le (29i) ou les canaux de sortie (29i, 29a) sont portés par des supports (27) s'étendant dans un plan perpendiculaire à un plan du boîtier pour être surélevés par rapport aux canaux d'entrée (23i, 23a, 25).

9. Cassette selon la revendication 7 ou 8, **caractérisée en ce que** le boîtier (21) est pourvu d'une voie de communication (28) entre le canal de sortie (29i) communiquant avec la tubulure d'irrigation (1 i) et le canal d'entrée (23a) communiquant avec la tubulure d'aspiration (1a) ou le troisième canal d'entrée (25) monté en dérivation par rapport à ce dernier.

10. Cassette selon la revendication 9, **caractérisée en ce que** la voie de communication (28) est assurée par une tubulure disposée dans une chambre (43) et à distance d'une paroi de fond (45) de cette chambre pour être comprimée contre cette paroi de fond dans une position d'obstruction de cette voie de communication.

11. Cassette selon l'une quelconque des revendications 1 à 10, **caractérisée en ce que** le support (5), le guide en T (13) avec le double arrondi (17) à la tête du T et le logement (21) du corps du T, le boîtier intégré au support (21) pourvu du (23i) ou des canaux d'entrée (23i, 23a), le troisième canal d'entrée (25), la (3i) ou les prises d'entrée (3i, 3a), la (7i) ou les prises de sortie (7i, 7a) et les supports (27) sont d'une seule pièce moulée par injection de matière plastique.

## Claims

1. A cassette intended to be inserted into an irrigation or aspiration machine used in endoscopy comprising an irrigation tube (1i) or an aspiration tube (1a) and a support (5) furnished with one (3i) or with two inlet plugs (3i, 3a), and with one (7i) or with two outlet plugs (7i, 7a), the tube or the two tubes forming an elbow (9i, 9a) for engaging with the inlet and outlet plug or the two inlet and outlet plugs in a respectively incoming (E) and outgoing (S) direction of flow and forming a segment of irrigation (11i) or of aspiration (11a) pumping in the incoming direction (E) of flow, **characterized in that** the support (5) comprises a T guide (13) shaped to the head of the T so as to protect the elbow (9i, 9a) of each tube (1i, 1a) and shaped along the body of the T as a slot (19) guiding the tube or the two tubes in the outgoing direction of flow (S), the T guide (13) running between the inlet plug or the two inlet plugs (3i, 3a) so as to form the segment of irrigation (11i) or of aspiration (11a) pumping on either side of the slot (19) between each inlet plug (3i, 3a) and the head of the T.

2. The cassette as claimed in claim 1, **characterized in that** the head of the T comprises a protective hood (14) for the elbow (9i, 9a) of each tube.

3. The cassette as claimed in claim 1 or 2, **characterized in that** the head of the T comprises a double rounding (17) for guiding the elbow (9i, 9a) of each tube.

4. The cassette as claimed in claim 1, **characterized in that** the T guide (13) is fixed to a housing (21) integrated with the support (5) and provided with one (23i) or with two inlet channels (23i, 23a) open at an inlet end (15i, 15a) and emerging at an opposite end via the inlet plug (3i) or the inlet plugs (3i, 3a) so as to ensure communication with the tube (1i) or the two tubes (1i, 1a) in the incoming direction of flow (E).

5. The cassette as claimed in claim 4, **characterized in that** the housing (21) is provided with a third inlet channel (25) open at one end (26) and disposed bypass-wise with respect to the inlet channel (23a) communicating with the aspiration tube (1a) so as to emerge, at an opposite end, via the inlet plug (3a) ensuring communication with the aspiration tube (1a).

6. The cassette as claimed in claim 5, **characterized in that** the inlet channel (23a) communicating with the aspiration tube (1a) and the third inlet channel (25) mounted bypass-wise open out, at the opposite end to the inlet plug (3a) ensuring communication with the aspiration tube (1a), into a chamber (31) integrated with the housing and receiving two complementary aspiration tubes (33, 35) engaging with these two channels (23a, 25) while being disposed some distance from a back wall (36) of the chamber (31) so as to be compressed against this back wall (36) in a position of obstruction of this inlet channel (23a) communicating with the aspiration tube (1a) or of this third inlet channel (25).

7. The cassette as claimed in claim 4, 5 or 6, **characterized in that** the housing (21) is provided with one (29i) or with two outlet channels (29i, 29a) open at an outlet end and emerging at an opposite end via the outlet plug (7i) or the outlet plugs (7i, 7a) so as to ensure communication with the tube (1i) or the two tubes (1i, 1a) in the outgoing direction of flow (S).

8. The cassette as claimed in claim 7, **characterized in that** the outlet channel (29i) or the outlet channels (29i, 29a) are carried by supports (27) extending in a plane perpendicular to a plane of the housing so as to be raised up with respect to the inlet channels (23i, 23a, 25).

9. The cassette as claimed in claim 7 or 8, **characterized in that** the housing (21) is provided with a communication pathway (28) between the outlet channel (29i) communicating with the irrigation tube (1i) and the inlet channel (23a) communicating with the aspiration tube (1a) or the third inlet channel (25) mounted bypass-wise with respect to the latter.

10. The cassette as claimed in claim 9, **characterized in that** the communication pathway (28) is ensured by a tube disposed in a chamber (43) and some distance from a back wall (45) of this chamber so as to be compressed against this back wall in a position of obstruction of this communication pathway.

11. The cassette as claimed in any one of claims 1 to 10, **characterized in that** the support (5), the T guide (13) with the double rounding (17) at the head of the T and the slot (21) of the body of the T, the housing integrated with the support (21) provided with the inlet channel (23i) or with the inlet channels (23i, 23a), the third inlet channel (25), the inlet plug (3i) or the inlet plugs (3i, 3a), the outlet plug (7i) or the outlet plugs (7i, 7a) and the supports (27) are in one piece of injection-molded plastic.

## Patentansprüche

1. Kassette zum Einsetzen in ein in der Endoskopie verwendetes Gerät für die Irrigation oder Aspiration mit einem Irrigationsrohr (1i) oder einem Aspirationsrohr (1a) sowie einem Träger (5), der mit einem (3i) oder zwei Eintrittsanschlüssen (3i, 3a) und mit einem (7i) oder zwei Austrittsanschlüssen (7i, 7a) versehen ist, wobei das Rohr bzw. die beiden Rohre einen Rohrbogen (9i, 9a) bilden, um in einer Richtung des Eintritts- (E) bzw. Austritts- (S) kreislaufs mit einem oder zwei Eintritts- und Austrittsanschlüssen in Eingriff zu gelangen, und wobei sie weiter in der Richtung des Eintritts- (E) kreislaufs einen Pumpabschnitt für die Irrigation (11i) oder Aspiration (11a) bilden, **dadurch gekennzeichnet, dass** der Träger (5) eine T-förmige Führung (13) umfasst, die am Kopfstück des T so gestaltet ist, dass sie den Rohrbogen (9i, 9a) jedes Rohres (1i, 1a) schützt, und die am Rumpf des T entlang zu einem Lager (19) geformt ist, das das bzw. die beiden Rohre in der Richtung des Austritts- (S) kreislaufs führt, wobei sich die T-förmige Führung (13) zwischen dem oder den beiden Eintrittsanschlüssen (3i, 3a) erstreckt, um den Pumpabschnitt für die Irrigation (11i) oder Aspiration (11a) zu beiden Seiten des Lagers (19) zwischen jedem Eintrittsanschluss (3i, 3a) und dem Kopf des T zu bilden.

2. Kassette nach Anspruch 1, **dadurch gekennzeichnet, dass** der Kopf des T eine Schutzkappe (14) für den Rohrbogen (9i, 9a) jedes Rohres umfasst.

3. Kassette nach Anspruch 1 oder 2, **dadurch gekennzeichnet, dass** der Kopf des T eine doppelte Rundung (17) umfasst, um den Rohrbogen (9i, 9a) jedes Rohres zu führen.

4. Kassette nach Anspruch 1, **dadurch gekennzeichnet, dass** die T-förmige Führung (13) an einem mit dem Träger (5) integral ausgebildeten Gehäuse (21) befestigt und mit einem (23i) oder zwei Eintrittskanälen (23i, 23a) versehen ist, die an einem Eintrittsende (15i, 15a) offen sind und an einem entgegengesetzten Ende in den (3i) oder die Eintrittsanschlüsse (3i, 3a) münden, um eine Verbindung mit dem (1i) oder den beiden Rohren (1i, 1a) in der Richtung des Eintrittskreislaufs (E) zu gewährleisten.

5. Kassette nach Anspruch 4, **dadurch gekennzeichnet, dass** das Gehäuse (21) mit einem dritten Eintrittskanal (25) versehen ist, der an einem Ende (26) offen und zum Eintrittskanal (23a), der mit dem Aspirationsrohr (1a) in Verbindung steht, parallel angeordnet ist, um an einem entgegengesetzten Ende in den Eintrittsanschluss (3a) zu münden, der die Verbindung mit dem Aspirationsrohr (1a) gewährleistet.

6. Kassette nach Anspruch 5, **dadurch gekennzeichnet, dass** der mit dem Aspirationsrohr (1a) in Verbindung stehende Eintrittskanal (23a) und der parallel angeschlossene dritte Eintrittskanal (25) an dem Ende, das zum Eintrittsanschluss (3a), der die Verbindung mit dem Aspirationsrohr (1a) gewährleistet, entgegengesetzt ist, in eine Kammer (31) münden, die mit dem Gehäuse integriert ist und zwei zusätzliche Aspirationsrohre (33, 35) aufnimmt, die mit diesen beiden Kanälen (23a, 25) im Eingriff stehen, während sie vom Boden (36) der Kammer (31) beabstandet sind, um in einer Verschlussstellung dieses mit dem Aspirationsrohr in Verbindung stehenden Eintrittskanals (23a) oder dieses dritten Eintrittskanals (25) gegen diesen Boden (36) zusammengedrückt zu werden.

7. Kassette nach Anspruch 4, 5 oder 6, **dadurch gekennzeichnet, dass** das Gehäuse (21) mit einem (29i) oder zwei Austrittskanälen (29i, 29a) versehen ist, die an einem Austrittsende offen sind und an einem entgegengesetzten Ende in den (7i) oder die Austrittsanschlüsse (7i, 7a) münden, um eine Verbindung mit dem (1i) oder den beiden Rohren (1i, 1a) in der Richtung des Austrittskreislaufs (S) zu gewährleisten.

8. Kassette nach Anspruch 7, **dadurch gekennzeichnet, dass** der (29i) oder die Austrittskanäle (29i, 29a) von Trägern (27) gehalten werden, die sich in einer zu einer Ebene des Gehäuses senkrechten Ebene erstrecken, um bezüglich der Eintrittskanäle (23i, 23a, 25) erhöht zu sein.

9. Kassette nach Anspruch 7 oder 8, **dadurch gekennzeichnet, dass** das Gehäuse (21) mit einem Verbindungsweg (28) zwischen dem Austrittskanal (29i), der mit dem Irrigationsrohr (1i) in Verbindung steht, und dem Eintrittskanal (23a), der mit dem Aspirationsrohr (1a) in Verbindung steht, oder dem dritten Eintrittskanal (25), der parallel zu letzterem angeschlossen ist, versehen ist.

10. Kassette nach Anspruch 9, **dadurch gekennzeichnet, dass** der Verbindungsweg (28) durch ein Rohr gewährleistet ist, das in einer Kammer (43) und vom Boden (45) dieser Kammer beabstandet angeordnet ist, um in einer Position des Verschlusses dieses Verbindungsweges gegen diesen Boden zusammengedrückt zu werden.

11. Kassette nach einem der Ansprüche 1 bis 10, **dadurch gekennzeichnet, dass** der Träger (5), die T-förmige Führung (13) mit der doppelten Rundung (17) am Kopf des T und das Lager (21) des Rumpfes des T, das in den Träger (21) integrierte und mit dem (23i) oder den Eintrittskanälen (23i, 23a) versehene Gehäuse, der dritte Eintrittskanal (25), der (3i) oder die Eintrittsanschlüsse (3i, 3a), der (7i) oder die Austrittsanschlüsse (7i, 7a) und die Träger (27) aus einem einzigen, durch Kunststoffspritzen hergestellten Teil bestehen.
